# EUROPÄISCHE PATENTANMELDUNG

(11) **EP 2 065 036 A1**
(43) Veröffentlichungstag der Anmeldung: **03.06.2009**
(21) Anmeldenummer: 07022973.7
(22) Anmeldetag: 27.11.2007
(51) Int. Cl.: A61K 9/48, A23L 1/29, A23L 1/303, A23L 1/305, A61K 31/27

(54) **Vitaminpräparat**

(71) Anmelder: Nutri Team GmbH, 82377 Penzberg (DE)
(72) Erfinder: Lautenschläger, Tanja, 24147 Klausdorf, Schwentine (DE)
(74) Vertreter: Maiwald Patentanwalts GmbH

(57) **Zusammenfassung**

Zwei-Phasen-Präparat zur zeitlichen abgestuften Anwendung, umfassend
(a) eine Phase (A) umfassend wasserlösliche Vitamine und/oder die dazugehörigen Provitamine, wobei Phase (A) keine fettlöslichen Vitamine oder Provitamine zu den fettlöslichen Vitaminen enthält,
(b) eine Phase (B) umfassend Vitamine und/oder die dazugehörigen Provitamine, wobei die Anzahl der fettlöslichen Vitamineverbindungen größer ist als die Anzahl der wasserlöslichen Vitaminverbindungen.

## Beschreibung

Die vorliegende Erfindung betrifft ein neues Vitaminpräparat sowie dessen Verwendung.

Es ist allgemein bekannt, dass die Nährstoffe Kohlenhydrate sowie die Nahrungsfette vorrangig zur Deckung des Energiebedarfs dienen. Eiweiße sind ein wichtiger Aufbaustoff für Zellen und körpereigene Wirkstoffe, wie Enzyme und bestimmte Hormone. Zunehmend gewinnen Vitamine Mineralstoffe und Spurenelemente für die Gesundheit und Leistungsfähigkeit an Bedeutung.

Vitamine, Mineralstoffe und Spurenelemente sind lebensnotwendige Regelstoffe im Stoffwechsel und Schutznährstoffe für die Gesundheit. Durch die heutigen Lebensumstände bestehen große Gefahren bezüglich einseitiger Ernährung sowie die Bevorzugung von kalorienreichen Lebensmittel mit einer niedrigen Nährstoffdichte, was teilweise zu einer schlechten Versorgung des Körpers bezüglich Vitamine, Mineralstoffe und Spurenelementen führen kann. Hinzu kommen heutzutage auch die Problematiken aus Umweltbelastung erhöhten Alkohol- und Rauchkonsum sowie Stresssituation im Alltagsleben, wodurch ein erhöhter Bedarf an Vitaminen, Mineralstoffen und Spurenelementen besteht.

Vitamine sind lebensnotwendige Nahrungsbestandteile, die für die normale Funktion von menschlichen Organismen mehr oder weniger obligat und bedarfsgerecht zuzuführen sind, da sie nur aus äußeren Quellen bzw. unter dem Einfluss von Milieufaktoren, wie z. B. der Darmflora, zugänglich sind. Ihre spezifische biokatalytische Wirkung beruht auf Ersatz, der dem metabolischen Verschleiß unterliegenden Wirkgruppen von Enzymen. Von wissenschaftlicher Seite her ist bekannt, dass Vitamine der B-Gruppe als Coenzyme am intermediären Stoffwechsel beteiligt sind und die Vitamine C, E und Betacarotin vor allem als Antioxidanzien wirken. Ein Mangel infolge ungenügender Zufuhr oder Resorption, Störung der Darmflora oder Metabolismus, Anti-Vitamin-Wirkung oder gegenseitiger Verbrauch führen zu Hypo- und Avitaminosen.

Es sind zahlreiche Vitaminpräparate im Handel erhältlich. Diese Vitaminpräparate weisen eine Vielzahl von unterschiedlichen Vitaminen auf und sollen das Leistungsvermögen und die Regeneration fordern bzw. unterstützen. Allerdings sind die angebotenen Präparate in ihrer Zusammensetzung nicht ausgewogen, d. h. die einzelnen Bestandteile der Präparate wirken nicht unterstützend sondern kompetitiv, so dass die positive Wirkung der einzelnen Wirkstoffelemente nicht eintritt.

Aus diesem Grunde ist es Aufgabe der vorliegenden Erfindung ein Präparat bereitzustellen, insbesondere welches sich als Nahrungsergänzungsmittel eignet, welches die Behinderung der einzelnen Komponenten ausschließt oder mindert und so die bessere Zufuhrung oder Aufnahme der einzelnen Nährstoffe, insbesondere Vitamine, im menschlichen Körper fördert.

Die Erkenntnis der vorliegenden Erfindung besteht darin, ein Präparat bereitzustellen, welches zwei unterschiedliche Phasen aufweist, die Phasen jeweils unterschiedliche Vitamine beinhalten, und diese beiden Phasen zeitlich abgestuft angewendet werden können, so dass negative Wechselwirkungen zwischen den einzelnen Vitaminen ausgeschlossen werden können.

Demnach richtet sich die vorliegende Erfindung in einem Aspekt auf ein Zwei-Phasen-Präparat zur zeitlich abgestuften Anwendung umfassend
(a) eine Phase (A) umfassend wasserlösliche Vitamine und/oder die dazugehörigen Provitamine, wobei Phase (A) keine fettlöslichen Vitamine oder Provitamine zu den fettlöslichen Vitaminen enthält,
(b) eine Phase (B) umfassend Vitamine und/oder die dazugehörigen Provitamine, wobei die Anzahl der fettslöslichen Vitaminverbindungen größer ist als die Anzahl der wasserlöslichen Vitaminverbindungen.

Vorzugsweise enthält Phase (B) nur wasserlösliche Vitamine die nicht Bestandteil der Phase (A) sind.

In einer zweiten Ausführungsform richtet sich die vorliegende Erfindung auf ein Kit zur zeitlichen abgestuften Anwendung, wobei das Kit umfasst
(a) eine Phase (A) umfassend wasserlösliche Vitamine und/oder die dazugehörigen Provitamine, wobei Phase (A) keine fettlöslichen Vitamine oder Provitamine zu den fettlöslichen Vitaminen enthält,
(b) eine Phase (B) umfassend Vitamine und/oder die dazugehörigen Provitamine, wobei die Anzahl der fettlöslichen Vitaminverbindungen größer ist als die Anzahl der wasserlöslichen Vitaminverbindungen.

Vorzugsweise enthält Phase (B) nur wasserlösliche Vitamine die nicht Bestandteil der Phase (A) sind.

Im Folgenden werden bevorzugte Merkmale der beiden oben bezeichneten Ausführungsformen gemeinsam genauer beschrieben.

Vorzugsweise sind die Phasen (A) und (B) unterschiedlich ausgestaltet, d. h. weisen eine unterschiedliche Form- und/oder Farbgebung auf. Es ist insbesondere bevorzugt, dass beide Phasen sich durch unterschiedliche Farben auszeichnen. So ist es insbesondere bevorzugt, dass die Phase (A) rot oder orange ist und/oder die Phase (B) blau ist.

Der Vorteil von unterschiedlicher Form- und/oder Farbgebung ist es, dass der Verbraucher die beiden Phasen leicht voneinander unterscheiden kann.

Bei den Phasen (A) und (B) handelt es sich insbesondere um Kapseln. Insbesondere sind Gelatinekapseln bevorzugt. Denkbar ist auch, dass es sich bei den Phasen (A) und (B) um Tabletten, Dragees, Granulate, Brausetabletten, Pulver, Trinklösungen oder andere übliche Darreichungsformen handelt.

Es hat sich herausgestellt, dass solch ein Zwei-Phasen-Präparat es ermöglicht, dass die Vitamine besonders günstig vom Körper aufgenommen werden, da eine gegenseitige Behinderung (z.B. Komplexbildung) bestimmter Vitamintypen insbesondere zwischen fettlöslichen und wasserlöslichen Vitaminen sowie bestimmten Mineralstoffen und Spurenelementen unterbunden werden kann.

Wichtig ist für die vorliegende Erfindung, dass die beiden Phasen (A) und (B) zeitlich abgestuft Anwendung finden, d. h. nicht gleichzeitig eingenommen werden. Insbesondere hat es sich als besonders positiv herausgestellt, dass die Phasen (A) und (B) 24 Stunden versetzt zueinander angewendet werden.

Demnach ist es insbesondere vorteilhaft, wenn
(a) die Phase (A) zu den Mahlzeiten an den Tagen X + 2n und
(b) die Phase (B) zu den Mahlzeiten an den Tagen X + (2n+1) angewendet werden, wobei "n" die nichtnegativen ganzen Zahlen repräsentiert, d. h. 0, 1, 2, 3, 4 usw.

"X" steht für einen beliebigen Tag, insbesondere ist "X" der Tag an dem die Aufnahme des Präparats oder des Kits begonnen wird.

In einer besonderen Ausführungsform werden die Phasen (A) und (B) zum Frühstück oder Abendessen eingenommen und zwar an alternierenden Tagen.

Im Folgenden soll näher auf die einzelnen Phasen eingegangen werden.

Entscheidend ist, dass die Phasen (A) und (B) unterschiedliche Vitamine enthalten, so dass gewährleistet ist, dass eine gegenseitige Behinderung der einzelnen Komponenten unterbleibt.

Demnach umfasst Phase (A) wasserlösliche Vitamine und/oder die dazugehörigen Provitamine, wobei Phase (A) keine fettlöslichen Vitamine oder Provitamine zu den fettlöslichen Vitaminen enthält.

Bezüglich dem Ausdruck "Provitamine" sei angemerkt, dass es sich dabei um Vorstufen der Vitamine handelt und diese erst noch in die entsprechenden Vitamine umgewandelt werden müssen, damit sie dem Körper von Nutzen sein können. So wird z. B. das Provitamin zu Vitamin D (7-Dehydrocholesterol) in der Haut durch UV-Strahlung z. B. sonnenlicht in das Vitamin D (Cholecalciferol) umgewandelt. β-Carotin wiederum stellt das Provitamin des Retinol (Vitamins (A)) dar.

Unter Vitamine werden alle organischen Verbindungen verstanden, die der menschliche Organismus nicht als Energieträger, sondern für andere lebenswichtige Funktionen benötigt, die jedoch der Stoffwechsel zum größten Teil nicht synthetisieren kann. Demnach müssen Vitamine mit der Nahrung aufgenommen werden.

Bei den wasserlöslichen Vitaminen handelt es sich um Thiamin (Vitamin B₁), Riboflavin (Vitamin B₂), Nicotinamid (Vitamin B₃), Pantothensäure (Vitamin B₅), Pyridoxin (Vitamin B₆), Biotin (Vitamin H), Folsäure (Vitamin B₉) und Cobalamin (Vitamin B₁₂). Demnach ist es insbesondere erstrebenswert, dass die Phase (A) zumindest drei dieser Vitamine enthält, bevorzugterweise jedoch fünf noch mehr bevorzugt alle wasserlöslichen Vitamine, wie oben genannt.

Bezüglich Phase (B) ist es erstrebenswert, dass diese Phase hauptsächlich fettlösliche Vitamine und/oder deren dazugehörigen Provitamine enthält. Jedoch ist es denkbar, dass Phase (B) auch andere Vitamine, nämlich wasserlösliche Vitamine und/oder deren Provitamine umfasst. Es muss die Anzahl der fettlöslichen Vitaminverbindungen und/oder deren Provitamine größer sein als die Anzahl der wasserlöslichen Vitaminverbindungen und/oder deren Provitamine in Phase (B). In einer besonderen Ausführungsform umfasst Phase (B) insbesondere Vitamine mit anitoxidanter Wirkung. Demnach ist es bevorzugt, dass in Phase (B) zumindest die fettlöslichen Vitamine Retinol (Vitamin (A)) und/oder dessen Provitamin Betacarotin, Tocopherol (Vitamin E) und Calciferol (Vitamin D) sowie als wasserlösliches Vitamin Ascorbinsäure (Vitamin C) umfasst. Bevorzugt enthält Phase (B) als Vitamine ausschließlich Retinol (Vitamin (A)) und/oder dessen Provitamin Betacarotin, Tocopherol (Vitamin E), Calciferol (Vitamin D) und Ascorbinsäure (Vitamin C).

Natürlich können in der Phase (B) noch weitere fettlösliche Vitamine vorhanden sein. Demnach ist es insbesondere erstrebenswert, dass zumindest drei fettlösliche Vitamine ausgewählt aus der Gruppe bestehend aus Retinol (Vitamin (A)), Calciferol (Vitamin D), Tocopherol (Vitamin E), Phyllochinon (Vitamin K₁), Menachinon (Vitamin K₂) und deren Provitamine in Phase (B) enthalten sind. Besonders bevorzugte fettlösliche Vitamine sind die eingangs erwähnten Tocopherol (Vitamin E), und Retinol (Vitamin (A)) bzw. dessen Provitamin Betacarotin, sowie Cholecalciferol (Vitamin D).

Darüber hinaus ist es denkbar, dass Phase (B) zwei wasserlösliche Vitamine enthält, wobei vorzugsweise mindestens eins davon Ascorbinsäure (Vitamin C) ist. Es ist insbesondere bevorzugt, dass als wasserlösliches Vitamin Phase (B) nur Ascorbinsäure (Vitamin C) enthält.

Denkbar ist auch, dass die Phasen (A) und (B) zusätzlich zu den Vitaminen weitere Mineralien bzw. Spurenelemente aufweisen. Demnach enthält ein Zwei-Phasen-Präparat oder ein Kit der vorliegenden Erfindung in der Phase (A) zusätzlich Magnesium oder Zink und/oder Grüntee.

Die Phase (B) des Zwei-Phasen-Präparates oder des Kits kann vorzugsweise zusätzlich Zinkglukonat und/oder Traubenschalenextrakt umfassen.

Es hat sich herausgestellt, dass insbesondere die Trennung von Zink und Magnesium einen positiven Effekt auf die Wirkung auf den menschlichen Organismus hat.

Die Mengen der einzelnen Vitamine hängen von unterschiedlichen Faktoren, d. h. ob die Person z. B. unter erhöhtem Stress oder möglichen Krankheiten leidet. Je nach Bedarf kann die entsprechende Menge bestimmt werden. Jedoch hat sich herausgestellt, dass es besonders bevorzugt ist, wenn die Phase (B) 10 bis 45 mg Betacarotin, 15 bis 90 mg Tocopherol (Vitamin E), 0,001 bis 0,009 mg Cholecalciferol (Vitamin D), 150 bis 540 mg Ascorbinsäure (Vitamin C), optional 50 bis 130 mg Zinkglukonat, und optional 60 bis 120 mg Traubenschalenextrakt umfasst.

Bezüglich Phase (A) hat es sich als bevorzugt herausgestellt, dass diese 0,1 bis 0,9 mg Biotin (Vitamin H), 0,2 bis 1,2 mg Folsäure (Vitamin B₉), 2,0 bis 12,0 mg Thiaminmononitrat (Vitamin B₁), 2,0 bis 12,0 mg Riboflavin-5-phosphat (Vitamin B₂), 5,0 bis 45,0 mg Nicotinamid (Vitamin B₃), 5,0 bis 45,0 mg Calcium D-Pantothenat (Vitamin B₅), 3,0 bis 18,0 mg Pyridoxin HCl (Vitamin B₆), 0,001 bis 0,01 mg Cyanocobalamin (Vitamin B₁₂), optional 200 bis 700 mg Magnesiumcarbonat oder 200 bis 700 mg Magnesiumcitrat, optional 0,03 bis 0,2 mg Natriumselenat oder 60 bis 300 mg reines Magnesium und optional 20 bis 240 mg Grüntee umfasst.

Das in dieser Erfindung beschriebene Zwei-Phasen-Präparat bzw. das dazugehörige Kit hat sich insbesondere als Nahrungsergänzungsmittel bewährt. Demnach richtet sich die vorliegende Erfindung auch auf Nahrungsergänzungsmittel, die das Zwei-Phasen-Präparat umfassen sowie Nahrungsergänzungsmittel die aus dem beschriebenen Kit bestehen. Auch richtet sich die vorliegende Erfindung auf eine Verwendung einer Phase (A) und einer Phase (B) wie in der vorliegenden Erfindung beschrieben zur Herstellung eines Zwei-Phasen-Präparates oder eines Kits als Nahrungsergänzungsmittel.

Die vorliegende Erfindung wird im Folgenden genauer durch folgende Beispiele beschrieben.

### Beispiele

Als besonders geeignet hat sich folgendes Zwei-Phasen-System bewert

### Blaue Kapsel:

| | |
|---|---|
| Vitamin H (Biotin) 0,3 mg | 200 % |
| Vitamin B₉ (Folsäure) 0,4 mg | 200 % |
| Vitamin B₁ (Thiaminmononitrat) 4,0 mg | 286 % |
| Vitamin B₂ (Riboflavin-5-phosphat) 4,0 mg | 250 % |
| Vitamin B₃ (Nicotinsäureamid) 15 mg | 83,3 % |
| Vitamin B₅ (Pantothensäure als Calcium-D-pantothenat) 15 mg | 250 % |
| Vitamin B₆ (Pyridoxin HCl 100 %) 6,0 mg | 300 % |
| Vitamin B₁₂ (Cyanocobalamin) 0,002 mg | 200 % |
| Magnesiumcarbonat 240 mg | 20 % |
| (entspricht 60,0 mg Magnesium) | |
| Natriumselenat 0,2 mg | *** |
| (entspricht 0,084 mg Selen) | |
| Grüntee 80 mg (95 %) | *** |

### Orange Kapsel

| | |
|---|---|
| Betacarotin 15 mg | 150 % |
| (entspricht 1200 mcg Vitamin A) | |
| Vitamin C (Ascorbinsäure 97 %) 180 mg | 300 % |
| Vitamin E (Tocopherolacetat) 30 mg | 300 % |
| Vitamin D3 (Cholecalciferol) 0,005 mg | 100 % |
| Zinkglukonat 66,42 mg - 104 mg | |
| (entspricht 9 - 12 Zink) | |
| Traubenschalenextrakt 80 mg | *** |

| | |
|---|---|
| *** Kein RDA-Wert festgesetzt | |

## Patentansprüche

1. Zwei-Phasen-Präparat zur zeitlichen abgestuften Anwendung, umfassend
(a) eine Phase (A) umfassend wasserlösliche Vitamine und/oder die dazugehörigen Provitamine, wobei Phase (A) keine fettlöslichen Vitamine oder Provitamine zu den fettlöslichen Vitaminen enthält
(b) eine Phase (B) umfassend Vitamine und/oder die dazugehörigen Provitamine, wobei die Anzahl der fettlöslichen Vitamineverbindungen größer ist als die Anzahl der wasserlöslichen Vitaminverbindungen.

2. Zwei-Phasen-Präparat nach Anspruch 1, worin Phase (A) und Phase (B) 24 Stunden versetzt zueinander angewendet werden.

3. Zwei-Phasen-Präparat nach Anspruch 1 oder 2, worin
(a) die Phase (A) zu Mahlzeiten an den Tagen X + 2n, wobei "n" die nichtnegativen ganzen Zahlen repräsentiert und
(b) die Phase (B) zu Mahlzeiten an den Tagen X + (2n + 1), wobei "n" die nichtnegativen ganzen Zahlen repräsentiert,
angewendet werden.

4. Zwei-Phasen-Präparat nach Anspruch, worin die Mahlzeiten das Frühstück oder das Abendessen sind.

5. Zwei-Phasen-Präparat nach einem der vorhergehenden Ansprüche, worin die Phase (B) maximal zwei wasserlösliche Vitamine enthält.

6. Zwei-Phasen-Präparat nach einem der vorhergehenden Ansprüche, worin die Phase (B) zusätzlich Zinkglukonat und/oder Traubenschalenextrakt umfasst.

7. Zwei-Phasen-Präparat nach einem der vorhergehenden Ansprüche, worin die Phase (A) zusätzlich Magnesium oder Zink oder Grüntee umfasst.

8. Zwei-Phasen-Präparat nach einem der vorhergehenden Ansprüche, worin die Phase (B) zumindest drei fettlösliche Vitamine ausgewählt aus der Gruppe bestehend aus Retinol (Vitamin A), Cholecalciferol (Vitamin D), Tocopherol (Vitamin E), Phyllochinon (Vitamin K₁), Menachinon (Vitamin K₂) und deren Provitamine umfasst.

9. Zwei-Phasen-Präparat nach einem der vorhergehenden Ansprüche, worin die Phase (B) Ascorbinsäure (Vitamin C) als das einzige wasserlösliche Vitamin umfasst.

10. Zwei-Phasen-Präparat nach einem der vorhergehenden Ansprüche, worin die Phase (A) zumindest drei wasserlösliche Vitamine ausgewählt aus der Gruppe bestehend aus Ascorbinsäure (Vitamin C), Thiamin (Vitamin B₁), Riboflavin (Vitamin B₂), Nicotinamid (Vitamin B₃), Pantothensäure (Vitamin B₅), Pyridoxin (Vitamin B₆), Biotin (Vitamin H), Folsäure (Vitamin B₉) Cobalamin (Vitamin B₁₂) und deren Provitamine umfasst.

11. Zwei-Phasen-Präparat nach einem der vorhergehenden Ansprüche, worin die beiden Phasen unterschiedliche Farben aufweisen.

12. Zwei-Phasen-Präparat nach Anspruch 8, worin die Phase (B) orange oder rot ist.

13. Zwei-Phasen-Präparat nach Anspruch 8 oder 9, worin die Phase (A) blau ist.

14. Zwei-Phasen-Präparat nach einem der vorhergehenden Ansprüche, worin die beiden Phasen jeweils als unterschiedliche Kapseln ausgebildet sind.

15. Zwei-Phasen-Präparat nach einem der vorhergehenden Ansprüche, worin Phase (B) als Vitamine ausschließlich
(a) die fettlöslichen Vitamine Cholecalciferol (Vitamin D), Retinol (Vitamin (A)) und Tocopherol (Vitamin E) und
(b) das wasserlösliche Vitamin Ascorbinsäure (Vitamin C) enthält.

16. Zwei-Phasen-Präparat nach einem der vorhergehenden Ansprüche, worin Retinol (Vitamin (A)) zumindest teilweise durch das Provitamin β-Carotin ersetzt ist.

17. Zwei-Phasen-Präparat nach einem der vorhergehenden Ansprüche, worin Phase (A) als Vitamine ausschließlich die wasserlöslichen Vitamine Thiamin (Vitamin B₁), Riboflavin (Vitamin B₂), Nicotinamid (Vitamin B₃), Pantothensäure (Vitamin B₅), Pyridoxin (Vitamin B₆), Biotin (Vitamin H), Folsäure (Vitamin B₉) und Cobalamin (B₁₂) enthält.

18. Zwei-Phasen-Präparat nach einem der vorhergehenden Ansprüche, worin das Präparat eine Phase (A) und Phase (B) umfasst worin
(a) die Phase (B) eine orange oder rote Kapsel ist, die
(i) 10 bis 15mg β-Carotin
(ii) 15 bis 35 mg Tocopherol (Vitamin E)
(iii) 0.001 bis 0.009 mg Calciferol (Vitamin D)
(iv) 150 bis 200 mg Ascorbinsäure (Vitamin C)
(v) optional 50 bis 260 mg Zinkglukonat und/oder 15 bis 30 mg reines Zink und
(vi) optional 60 bis 120 mg Traubenschalenextrakt umfasst, und
(b) die Phase (A) eine blaue Kapsel ist, die
(i) 0.1 bis 0.9 mg Biotin (Vitamin H),
(ii) 0.2 bis 1.2 mg Folsäure (Vitamin B₉)
(iii) 2.0 bis 12.0 mg Riboflavin-5-phosphat (Vitamin B₂),
(iv) 5.0 bis 45.0 mg Nicotinamid (Vitamin B₃),
(v) 5.0 bis 45.0 Calcium D-Pantothenat (Vitamin B₅),
(vi) 3.0 bis 18.0 mg Pyridoxin HCl (Vitamin B₆),
(vii) 0.001 bis 0.03 mg Cyanocobalamin (Vitamin B₁₂),
(viii) optional 200 bis 300 mg Magnesiumcarbonat und/oder 60 bis 300 mg reines Magnesium
(ix) optional 0.05 bis 0.6 mg Natriumselenat und/oder 0.03 bis 0.2 mg reines Selen
(x) optional 20 bis 240 mg Grüntee umfasst.

19. Zwei-Phasen-Präparat nach einem der vorhergehenden Ansprüche als Nahrungsergänzungsmittel.

20. Kit umfassend die Phasen (A) und (B) nach einem der Ansprüche 1 bis 19.

21. Nahrungsergänzungsmittel nach einem der Ansprüche 1 bis 20.

22. Verwendung eines Zwei-Phasen-Präparat nach einem der Ansprüche 1 bis 19 oder eines Kits nach Anspruch 20 als Nahrungsergänzungsmittels.

23. Verwendung einer Phase (A) und einer Phase (B) nach einem der Ansprüche 1 bis 18 zur Herstellung eines zwei Phasen Präparates oder eines Kits als Nahrungsergänzungsmittel.
